Europäisches Patentamt

(19) European Patent Office

Office européen des brevets

(11) Numéro de publication: **0 235 484 B1**

(12) **FASCICULE DE BREVET EUROPEEN**

(45) Date de publication de fascicule du brevet:
24.07.91

(21) Numéro de dépôt: 86420061.3

(22) Date de dépôt: 24.02.86

(51) Int. Cl.⁵: **A61F 5/02**

(54) **Ceinture lombo-abdominale antalgique.**

(43) Date de publication de la demande:
09.09.87 Bulletin 87/37

(45) Mention de la délivrance du brevet:
24.07.91 Bulletin 91/30

(84) Etats contractants désignés:
**AT BE CH DE GB IT LI LU NL SE**

(56) Documents cités:
**DE-A- 2 506 647      FR-A- 755 689**
**FR-A- 809 069        FR-A- 2 114 033**
**US-A- 3 717 143      US-A- 4 470 417**

(73) Titulaire: **Bertheas, Michel**
**1, rue Jean Baptiste Marcet**
**F-42170 St Just St Rambert(FR)**

(72) Inventeur: **Bertheas, Michel**
**1, rue Jean Baptiste Marcet**
**F-42170 St Just St Rambert(FR)**

(74) Mandataire: **Dupuis, François**
**Cabinet Laurent et Charras 3, place de**
**l'Hôtel-de-Ville**
**F-42000 Saint-Etienne(FR)**

Rank Xerox (UK) Business Services

## Description

La présente invention concerne une ceinture lombo-abdominale antalgique selon le préambule de la revendication 1.

Les lombalgies dites communes, peuvent provenir d'un désordre disco-vertébral ou bien d'un désordre localisé sur les articulaires postérieures. Ches le sportif, ces désordres sont fréquemment en relation avec des contraintes mécaniques exagérées ou bien en relation avec des traumatismes directs (contusions...). Chez le sportif également, une lombalgie peut relever d'un traumatisme purement musculaire soit par surutilisation du secteur lombaire, soit par contusion.

Dans ces formes de lombalgie qui ne relèvent pas d'une pathologie organique évolutive et qui sont en règle générale passagères mais invalidantes, les massages et les compressions locales manuelles ou par bandage, exercés quelques minutes ou quelques heures, se révèlent souvent très antalgique et permettent en soulageant la douleur, de continuer la pratique sportive.

Selon l'art antérieur, on connait des ceintures de ce type composées d'une partie dorsale rigide, renforcée par des baleines ou présentant localement des excroissances aptes à venir en appui sur les régions lombaires et paralombaires. De telles ceintures ne donnent pas entière satisfaction car, en raison de leur rigidité, et selon leur mode de fixation, elles constituent un carcan immobilisant totalement le tronc et entravant le mouvement. De plus lorsque l'utilisateur s'incline vers l'avant, leurs extrémités s'éloignent du dos, tandis que leur partie centrale reste en appui sur la zone charnière du dos en risquant de blesser cette dernière.

On connait également par le brevet DE 2506647 et pour un problème posé et une application totalement différents un appareil destiné au soutien et à la correction de la colonne vertébrale. Cet appareil se présente sous la forme d'une ceinture élastique agencée avec un élément support de colonne vertébrale fixé amovible. Cet élément support est également agencé pour recevoir des corps amovibles susceptibles de venir en regard des vertèbres de la colonne vertébrale.

En pratique, bien que les différents éléments aient une certaine capacité de règlage transversal, l'appareil précité est utilisé par une application particulière et n'offre aucune diversité de positions d'utilisation en fonction de maux ou traitement différents à appliquer.

Le Brevet FR 2.114.033 décrit des éléments de ceinturage du corps agencés pour recevoir à position déterminée une pochette réalisée avec un filet ou une résille à l'intérieur de laquelle est introduit un morceau de peau de chat.

Le but recherché selon ce Brevet consiste essentiellement à la protection des éléments présentant des propriétés particulières et destinées à être appliquées contre le corps, et également à leur amovibilité pour permettre leur nettoyage ou lavage. Dans ces conditions, l'emplacement desdits éléments est prédéterminé.

On connaît aussi par le Brevet FR COTE 809069 la réalisation de ceintures abdominales agencées avec des éléments supports de moyens d'appui contre la peau. Bien que le produit résultant permette une application ponctuelle d'éléments d'appui, compte tenu de leurs dispositions et agencements sur des supports triangulés, leur positionnement est très limité, leur démontage peu pratique. En outre selon le but recherché, il s'agissait de répartir sur au moins trois points d'appui une force susceptible de refouler l'abdomen.

On observe également que les brevets FR 809069 et DE 2506647 réduisent très sensiblement la mobilité tronculaire.

Par ailleurs, on observe que les ceintures décrites selon l'art antérieur sont prévues pour un corps en position statique.

On connaît également par le Brevet US 4.470.417 une ceinture recevant un élément amovible et orientable pour s'adapter à l'emplacement de la partie de corps à traiter. Cet élément est une orthèse chauffante comprenant une pellicule pour exercer un effet de chaleur en des zônes considérées.

Le problème posé à l'origine de l'invention est totalement différent des objectifs recherchés dans les quatre brevets antérieurs précités.

La présente invention a pour but de fournir une ceinture lombo-abdominale qui permet de faire varier d'une manière très large les zones douloureuses du corps susceptibles d'être traitées par une orientation pluri-directionnelles instantanée et adaptée à toutes sortes de traumatismes des moyens d'appui, sans manipulation particulière et qui apporte également un complément antalgique sans antraver les mouvements du corps et permet à l'utilisateur de pratiquer ses activités courantes et mêmes certains sports.

La ceinture selon l'invention telle que caractérisée à la revendication 1 répond d'une part à l'effet de contention recherché, grâce au tissu élastique dont elle est composée. Elle correspond aussi à l'effet de compression douce maintenue grâce à l'existence des pelotes élastiques. Elle réalise d'autre part un massage doux mais insistant et permanent, de même que bien localisé sur les zones musculaires contracturées en regard du secteur vertébral qui souffre.

Enfin, grâce à ses propriétés élastiques et à son adaptabilité, elle ne gêne en aucune façon le mouvement nécessaire à l'activité sportive considérée.

D'autres caractéristiques et avantages ressortiront de la description qui suit en référence au dessin schématiquement annexé, représentant à titre d'exemples non limitatifs, une forme d'exécution de cette ceinture.

La figure 1 est une vue en plan par dessus, représentant la ceinture posé à plat ;

La figure 2 est une vue en perspective en coupe transversale montrant l'une des pelotes se rapportant sur la ceinture ;

La figure 3 est une vue partielle en plan par dessus montrant la disposition des pelotes sur la ceinture ;

La figure 4 est une vue schématique de côté montrant la ceinture en position d'utilisation.

Les figures 5 et 6 sont des vues en variantes à caractère schématique illustrant d'une manière non limitative, à titre d'exemple le positionnement des pelottes.

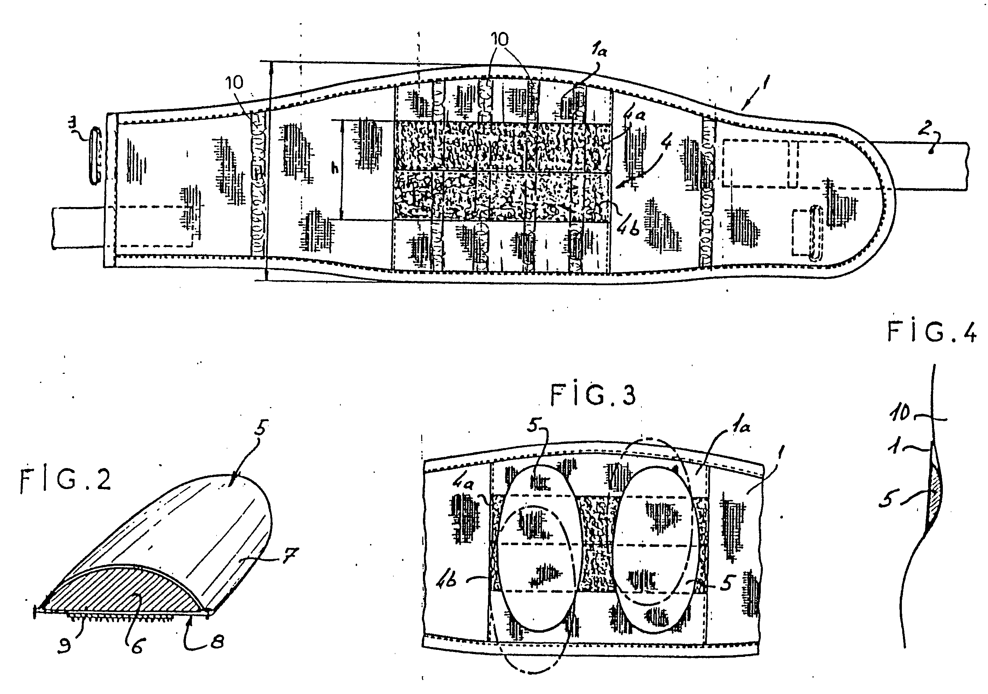

De façon connue, cette ceinture, destinée à venir, par sa partie dorsale, sur la région lombaire illio-costale, comme représenté à la figure 1 est composée par une bande dont les extrémités sont munies de moyens d'accrochage complémentaires, et, par exemple, de sangles (2) fixées à l'une des extrémités, et aptes à passer dans les anneaux (3) fixés à l'autre extrémité. Ces sangles sont par exemples réalisées en velours à crochets, de manière à pouvoir régler aisément la tension de serrage après rabattement de leurs extrémités ayant traversé la boucle (3).

Selon l'invention, au moins la partie postérieure de cette ceinture, partie s'étendant jusqu'aux flancs est réalisée dans un matériau tricoté ou tissé, et élastique transversalement et longitudinalement avec un fort indice d'élasticité assurant sur les masses musculaires lombaires, une pression permanente au repos et chez le sujet en mouvement, dont le but est d'assurer un effet antalgique. Cette ceinture élastique est renforcée sur la partie abdominale ainsi que sur la partie lombaire, et sa largeur est plus importante dans sa partie lombaire et sa partie abdominale. Elle est taillée de telle sorte qu'elle s'adapte au relief des crêtes iliaques et de l'angle ilio-lombaire assurant ainsi une meilleure adhérence au tronc. Elle comporte dans sa partie lombaire des ressorts plats (10) assurant une rigidité relative. La partie dorsale (1a) qui est plus large, comporte sur sa face antérieure des moyens d'accrochage (4) aptes à coopérer avec des moyens complémentaires portés par une ou plusieurs pelotes amovibles (5). Comme montré à la figure 2, chaque pelote, ayant la forme générale d'un disque bombé, elliptique ou toute autre forme appropriée, est composée d'un coussinet (6) en matière compressible élastique protégé par exemple et non limitativement, par une enveloppe en tissu (7). Sur sa face postérieure plane (8), chaque pelote comporte les moyens d'acrochage (9) aptes à coopérer avec les moyens d'accrochage (4) de la ceinture. Dans cette forme d'exécution, les moyens d'accrochage (4) et (9) sont constitués par des bandes de velours respectivement à boucles et crochets du type auto-agrippants.

La figure 1 met en évidence que les moyens d'accrochage (4) de la ceinture sont en fait, constitués par deux bandes de velours à boucles (4a) et (4b), disposées de façon symétrique sur cette même partie de chaque côté de l'axe vertébral repéré par les épineuses lombaires et également disposées sensiblement au milieu de la hauteur de la ceinture et s'étendant sur une hauteur (h) dont la valeur est supérieure au tiers de la hauteur (H) de la partie dorsale (1a) de la ceinture. Grâce à cette disposition, chaque pelote (5) peut être disposée dans n'importe quelle zone de la partie centrale et dorsale (1a) de la ceinture et être ainsi positionnée tant verticalement, suivant un axe parvertébral, qu'horizontalement, suivant un axe métamérique, qu'obliquement, de manière à coïncider parfaitement avec la zone douloureuse du corps.

Il résulte de ces dispositions que la ou les pelotes ont une capacité de positionnement pluridirectionnel instantané, et qu'elles peuvent déborder très largement de la zone comprenant les moyens d'accrochage (4) tout en conservant une partie nécessaire à l'accrochage en regard de ladite zone.

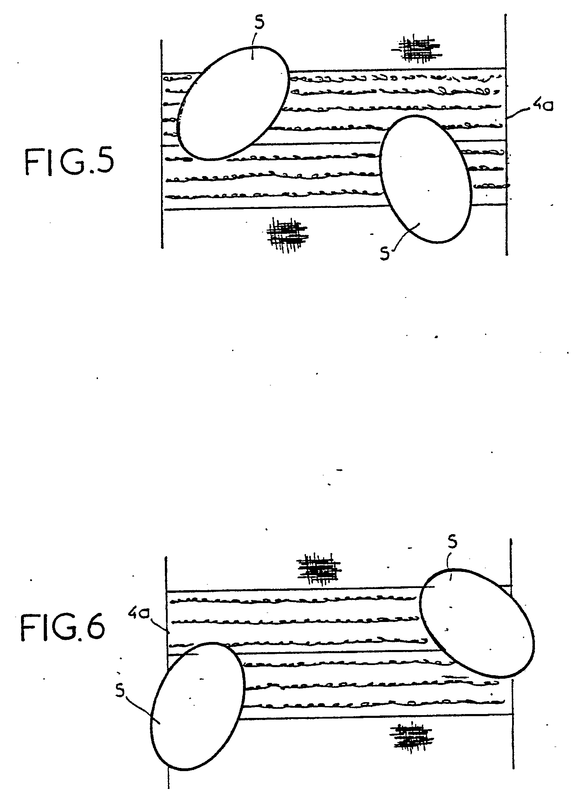

De plus grâce à cette amovibilité, il est possible, si besoin est, de n'utiliser qu'une pelote mais aussi de changer les pelotes de manière à les adapter tant par leur forme que par leur souplesse aux besoins de l'utilisateur et de les orienter en fonction des besoins , comme illustré par exemple et non limitativement aux figures 5 et 6.

Par ailleurs, à la partie abdominale de la ceinture et latéralement, un ressort plat flexible, assure une meilleure tenue de la ceinture élastique, en particulier lors des mouvements de flexion antérieure, latéro-flexion ou rotation du tronc, empêchant le tissu élastique de rouler sur lui-même.

Lorsque la ceinture est positionnée sur le corps (10), comme montré à la figure 4, la bande élastique (1) s'adapte intimement au tronc plaquant parfaitement sur les régions lombaires, para-lombaire et illio-costale et contient les muscles larges de l'abdomen en respectant la mobilité des dernières côtes et l'expansion de l'épigastre. La pression de placage se transmet à la zone douloureuse par le point sommital de chaque pelote (5). Cette pression, modérée mais constante, apporte à la contention lombaire un complément antalgique très appréciable et accélérant la disparition de la douleur. En outre, et grâce à sa structure élastique en tous sens, la ceinture suit les mouvements du corps sans pour autant apporter une gêne quelconque à ces derniers. Il en résulte que cette ceinture permet non seulement la pratique des activités courantes mais aussi celle de certains sports, ce qui n'est pas le cas avec les ceintures actuelles.

Cette ceinture offre par ailleurs les autres caractéristiques suivantes :

- Elle assure une bonne contention même au niveau abdominal sans pour autant comprimer les viscères.
- Elle ne gêne pas la fonction digestive car une fois mise en place, elle respecte l'aire épigastrique.
- La contention est règlable grâce au système bandes autoagrippantes du type Velcro-abdominal.
- La compression locale exercée par les pelotes sur les masses musculaires lombaires de chaque côté de l'axe vertébral est modulable de trois façons :
  . sur l'axe longitudinal,
  . dans un plan frontal,
  . dans une direction oblique,
  ce qui permet à chaque pelote d'être disposée de façon tout-à-fait indépendante par rapport à l'autre pelote, sur toute la face postérieure du secteur lombaire.
- L'ensemble du dispositif ne limite aucun mouvement du segment lombaire (flexion, extension, latéro-flexion droite, latéroflexion gauche, rotation droite, rotation gauche).

**Revendications**

1. Ceinture lombo-abdominale antalgique (1) comportant une zône plus large dans sa partie dorsale (1a) pour s'appliquer sur la région lombaire illio-costale et comportant, à ses extrémités antérieures, des moyens d'accrochage (2, 3) pour autoriser la fermeture de la ceinture et son maintien sur le corps, ladite ceinture (1) étant réalisée en matériau élastique transversalement et longitudinalement et présentant, sur la face intérieure de sa partie dorsale (1a), des moyens de fixation non élastiques (4) aptes à recevoir de manière amovible et en toute position sur leur surface des moyens de pression (5) aptes à appuyer sur une ou plusieurs parties du corps à soigner, ladite ceinture (1) étant caractérisée en ce que lesdits moyens de fixation (4) sont constitués par des bandes longitudinales non élastiques (4a, 4b) s'étendant sensiblement au milieu de la partie la plus large de la ceinture (1a) et sur plus d'un tiers de la hauteur de celle-ci, lesdits moyens de pression sont constitués par des pelotes (5) en matière compressible et élastique ayant la forme générale discoïde ou elliptique, lesdites pelotes (5) présentant une face postérieure (8) plane agencée avec des moyens de fixation (9) complémentaires des moyens de fixation (4) portés par la ceinture (1) et une face antérieure de forme bombée apte à exercer par la partie sommitale une pression modérée et localisée sur les zônes douloureuses du dos.

2. Ceinture selon la revendication 1, caractérisée en ce que lesdites bandes longitudinales (4a, 4b) fixées sur la face antérieure de la partie dorsale (1a) de la ceinture (1) sont constituées de velours à boucles et les moyens complémentaires de fixation (9) portés par la face postérieure (8) des pelotes (5) sont constitués par des bandes (9) réalisées en velours à crochets.

3. Ceinture selon l'une quelconque des revendications 1 et 2, caractérisée en ce que les pelotes (5) sont formées par un coussinet (6) en matériau compressible et élastique qui est protégé par une enveloppe en tissu (7), chaque pelote faisant partie d'une série de pelotes se diférenciant par la souplesse et la forme du coussinet.

4. Ceinture selon la revendication 1, caractérisée en ce qu'elle comporte dans sa partie lombaire des ressorts plats (10), et en ce que à la partie abdominale de ladite ceinture et latéralement est prévu un ressort plat flexible.

**Claims**

1. Antalgic lumbo-abdominal belt (1) having a wider area in its back portion (1a) for being applied to the ilio-costal lumbar region and provided at the forward ends thereof with hooking means (2, 3) for permitting the belt to be closed and held in place on the body of the patient, said belt (1) being made of transversely and longitudinally elastic material and being provided on the inside face of its back portion (1a) with non-elastic fastening means (4) capable of accommodating in a removable manner and in any position on their surface the pressure means (5) capable of pressing down one or more portions of the human body to be treated, said belt (1) being characterized in that said fastening means (4) consist of non-elastic longitudinal strips (4a, 4b) extending approximately in the middle of the broader portion of the belt (1a) and along more than one third of the height of said belt, said pressure means consisting of pads (5) of compressible and elastic material having a discoïdal or elliptical general shape, said pads (5) being formed with a rear face (8) provided with fastening means (9) complementary to the fastening means (4) supported by the belt (1), and with a front face having a dished shape capable of exercising through the apex portion a moderate and localized pressure on the sore areas of the back of the patient.

2. Belt as claimed in Claim 1, characterized in that said longitudinal strips (4a, 4b) secured to the front face of the dorsal portion (1a) of the belt (1) consist of of looped velvet, the complementary fastening means (9) supported by the rear face (8) of the pads (5) consisting of strips (9) made of hook velvet.

3. Belt as claimed in any one of Claims 1 and 2, characterized in that the pads (5) are formed by a cushion (6) of compressible and elastic material which is protected by a fabric covering (7), each pad forming a part of a series of pads differentiated from one another by the flexibility and the shape of the cushion.

4. Belt as claimed in Claim 1, characterized in that said belt is provided in the lumbar portion thereof with laminated springs (10), and in that a flexible laminated spring is provided laterally at the abdominal portion of said belt.

**Patentansprüche**

1. Antalgische Unterleibslendenbinde (1), die die in deren Rückenteil (1a) eine breitere Zone zum Anlegen auf der Hüftrippenlendenregion aufweist und an den Vorderenden die Einhakensmittel (2, 3) zum Schliessen und Festhalten der Binde auf dem Körper besitzt, wobei die genannte Binde (1) aus in Quer- sowie Längsrichtung federndem Werkstoff hergestellt ist und auf der Innenfläche deren Rückenteils (1a) nichtfedernde Befestigungsmittel (4) aufweist, die dazu geeignet sind, auf ihrer Oberfläche in abnehmbarer Weise und in beliebiger Stellung die zum Anpressen auf einem oder mehreren Teilen des zu behandelnden Körpers vorgesehenen Andrückmittel (5) aufzunehmen, indem die genannte Binde dadurch gekennzeichnet ist, dass die basagten Befestigungsmittel (4) aus nichtfedernden Längsstreifen (4a, 4b) bestehen, die sich annähernd in der Mitte des breitesten Teils der Binde (1a) und auf mehr als einem Drittel der Höhe dieser Binde erstrecken, und dass die besagten Andrückmittel durch aus eindrückbaren und elastischem Werkstoff bestehende Bälle (5) gebildet sind, die eine allgemeine diskoïdale oder ellipsoïdale Formgebung aufweisen, wobei die genannten Bälle (5) eine rückwärtige Planoberfläche (8) auch aufweisen, die mit den durch die Binde (1) getragenen Befestigungsmittel (4) ergänzenden Befestitungsmitteln (9) versehen ist, indem diese Bälle (5) eine gewölbte Vorderoberfläche aufweisen, die für das Ausüben durch deren Scheitelteil-eines gemässigten und lokalisierten Andrückens auf die schmerzhaften Zonen des Rückens vorgesehen ist.

2. Binde nach Anspruch 1, dadurch gekennzeichnet, dass die genannten, auf der Vorderfläche des Rückenteils (1a) der Binde (1) befestigten Längsstreifen (4a, 4b) aus lockigem Samt bestehen, wobei die durch die rückwärtige Oberfläche (8) der Bälle (5) getragenen, ergänzenden Befestigungsmittel (9) durch die aus Haken-samt bestehenden streifen (9) gebildet sind.

3. Binde nach einem der Ansprüche 1 und 2, dadurch gekennzeichnet, dass die Bälle (5) durch ein aus andrückbarem und federndem Werkstoff bestehendes Kissen (6) gebildet sind, das durch eine Umhüllung aus Gewebe (7) geschützt ist, wobei jeder Ball zu einer Reihe von Bällen gehört, die durch die Biegsamkeit und die Formgebung des Kissens unterschiedlich sind.

4. Binde nach Anspruch 1, dadurch gekennzeichnet, dass diese Binde in deren Lendenteil die Blatt-federn (10) aufweist, und dass eine biegsame Blattfeder an dem Unterleibsteil der genannten Binde und seitlich vorgesehen ist.

FIG. 4

FIG. 3

FIG. 2

6

FIG.5

FIG.6